# EUROPEAN PATENT SPECIFICATION

(11) **EP 3 193 858 B1**
(45) Date of publication and mention of the grant of the patent: **31.10.2018**
(21) Application number: 15756848.6
(22) Date of filing: 03.08.2015
(51) Int. Cl.: A61K 31/194, A61P 35/00

(54) **NEW MDR1 INHIBITORS FOR OVERCOMING MULTIDRUG RESISTANCE**
NEUE MDR1-INHIBITOREN ZUR ÜBERWINDUNG EINER MULTIPLEN MEDIKAMENTENRESISTENZ
NOUVEAUX INHIBITEURS MDR1 POUR SURMONTER LA RÉSISTANCE MULTIPLE AUX MÉDICAMENTS

(30) Priority: 04.08.2014 IT MI20141425
(43) Date of publication of application: 26.07.2017
(73) Proprietor: Università degli Studi di Parma, 43121 Parma (IT)
(72) Inventor: BERNINI, Franco, I-43123 Parma (IT); ADORNI, Maria Pia, I-43123 Parma (IT); PETRONINI, Pier Giorgio, I-43126 Parma (IT); ALFIERI, Roberta, I-43125 Parma (IT); GALETTI, Maricla, I-26034 Piadena (Cremona) (IT); FAVARI, Elda, I-29020 Gossolengo (Piacenza) (IT); INCERTI, Matteo, I-43122 Parma (IT)
(74) Representative: Gerli, Paolo
(86) International application number: PCT/EP2015/067795
(87) International publication number: WO 2016/020315

(56) References cited:
- WO-A1-93/21914
- WO-A1-2005/102985
- WO-A1-2007/135592
- WO-A2-99/01118
- MENG CHARLES Q ET AL: "Novel phenolic antioxidants as multifunctional inhibitors of inducible VCAM-1 expression for use in atherosclerosis.", BIOORGANIC & MEDICINAL CHEMISTRY LETTERS 16 SEP 2002, vol. 12, no. 18, 16 September 2002 (2002-09-16), pages 2545-2548, XP002732147, ISSN: 0960-894X
- PALMEIRA A ET AL: "Three decades of P-gp inhibitors: skimming through several generations and scaffolds.", CURRENT MEDICINAL CHEMISTRY 2012, vol. 19, no. 13, 2012, pages 1946-2025, XP002732148, ISSN: 1875-533X
- SANTOS SOFIA A ET AL: "Small molecule inhibitors of multidrug resistance gene (MDR1) expression: preclinical evaluation and mechanisms of action.", CURRENT CANCER DRUG TARGETS OCT 2013, vol. 13, no. 8, October 2013 (2013-10), pages 814-828, XP0009181115, ISSN: 1873-5576

## Description

### FIELD OF THE INVENTION

The present invention relates to the field of drugs, which are capable of opposing multidrug resistance, increasing the efficiency of therapies, particularly anti-cancer therapies, which are susceptible to these forms of resistance.

### BACKGROUND OF THE ART

Cell resistance to MDR drugs (*multi drug resistance*) is a complex phenomenon, which considerably limits the efficiency of pharmaceutical treatments. Such resistances are particularly frequent in the case of chemotherapeutic treatments. Some tumors are intrinsically resistant to pharmacological therapies, whereas other, which are initially sensitive to chemotherapy, become resistant during the treatment. Resistance to chemotherapeutic drugs may be caused by several factors of pharmacokinetic type, of metabolic type, associated to changes in the tumoral micro-environment, but it may especially depend on changes in the genic expression of tumor cells. Drug resistance depending on molecular alterations may reflect on the inactivation of the drug, on changes of the pharmacological target, on an enhanced DNA repair, on resistance to apoptosis, on uptake inhibition and on a higher outflow of the drug. An increased outflow of a drug from tumoral cells is mainly mediated by an overexpression of transporters which are located on the plasma membrane.

Among the membrane pumps, the most studied transporter, since it is most involved in the phenomenon of the resistance to chemotherapeutic drugs, is glycoprotein P (P-gp) or MDR1. MDR1 belongs to the family of the so-called *ATP-binding cassette* (ABC) transporters, and it mediates the active extrusion from the cells of a number of compounds, exploiting the ATP hydrolysis as an energy resource. When it is overexpressed, it considerably affects the bioavailability, distribution, and activity of many drugs having anti-cancer activity, making them ineffective. Furthermore, among all the pumps that cause resistance, the MDR1 transporter is characterized by the lowest specificity of substrate and the highest distribution at the level of tissues and organs, such as liver, intestines, brain and kidneys; all these aspect make the MDR1 protein more responsible for the phenomenon of the resistance to chemotherapeutic drugs. Its over-expression has been reported in a number of human tumors (leukemias/lymphomas, myelomas, breast, lung, ovary carcinomas). Molecules which are structurally very different can be actively extruded from the cell through the MDR1 activity, indicating that the resistance to an individual drug actually translates into a multi-drug resistance, which generally makes the chemotherapy ineffective.

The compounds interacting with the MDR1 receptor have been classified into three categories: substrates, inhibitors, and modulators. Substrates are molecules which are actively transported by the protein outside of the cell. Modulators modify the binding site of the substrate through a negative allosteric modulation. Finally, inhibitors block the translocation activity of MDR1 preventing the binding between ATP and the *nucleotide-binding domain* (NBD). A review of these compounds is the on-line publication Frontiers in Oncology, Jan. 2014, 4(2), Article 2.

A large number of studies tried to identify new and efficient strategies to oppose the phenomenon of the resistance to chemotherapeutic drugs, however with mediocre results. In fact, most of the so far developed inhibitors of the MDR1 transporter turned out to be not very active, or not very selective, and associated to several side effects.

First-generation inhibitors such as verapamil, quinine, and cyclosporine A showed to be toxic at the concentrations required for inhibiting MDR1. The cyclosporine D analogue, PSC-833, is a second-generation inhibitor which, however, showed pharmacokinetic interactions with unacceptable toxicity. However, also third-generation, high-affinity inhibitors and with low pharmacokinetic interactions (tariquidar, elacridar) showed a high toxicity profile when tested in association with chemoterapy (*Frontiers in Oncology,* op.cit.)

Some antioxidative compounds, e.g., probucol, were proposed for increasing resistance to anti-cancer drugs (see WO99/01118); however, such compounds are poorly specific towards the transporter MDR1, which also interfere with other transporters attending to important physiological functions useful for the patient: e.g., probucol is known for interfering with the transporter ABCA1, which physiologically attends to the trans-cell transport of cholesterol (Arterioscler.Thromb.Vasc.Biol. 2004 Dec;24(12):2345-50). The inhibition of these transporters alters the structure and operation of the cell, giving rise to possible pathologic phenomena. In particular, ABCA1 and ABCG1 are specifically involved in the reverse cholesterol transport (RCT), a physiological mechanism which promotes the clearance from the body of cholesterol excessively accumulated in the peripheral tissues, opposing the formation and development of atherosclerotic plaques. The first step of such process is the mechanism of outflow of free cholesterol from macrophages of the artery wall to lipoproteins circulating in the serum, such as HDLs and apolipoprotein A-I, and it is mediated by ABCA1 and ABCG1. This process is crucial for the maintenance of the homeostasis of cholesterol in the cells which, except for hepatocytes, are capable of getting cholesterol by the re-uptake of plasma lipoproteins or by the ex-novo synthesis, but they are not capable of catabolizing it. The inhibition of such pumps may promote an excessive accumulation of free intracellular cholesterol, which is known to trigger toxic events for the cell, up to cell death, concurring to the pathogenesis and development of the atherosclerotic disease (Westerterp M. et al. ATP-binding cassette transporters, atherosclerosis, and inflammation. Circ Res. 2014 Jan 3;114(1):157-70). The publication Bioorganic & Medicinal Chemistry Letters 12 (2002) 2545-48 discloses some phenolic antioxidants which are active inhibitors of the induction of VCAM-1 expression, proposed for the treatment of arteriosclerosis, and some biologically inactive intermediates which are used for the synthesis thereof.

The patent application WO93/21914 describes probucol diglutarate and probucol dimaleate compositions for treating atherosclerosis and reperfusion injury. The publication Bioorganic & Medicinal Chemistry Letters 2002, 12(18), 2545-2548 describe probucol disuccinate; the same compound is also disclosed in WO2005/102985, for use in treating atherosclerosis. The publications Current Medicinal Chemistry, 2012, 19(13), 1946-2025, Current Cancer Drug Targets, 2013, 13(8), 814-828 and WO2007/135592 describe a number of P-gp inhibitors as potential MRD reversers.

In the state of the art, the need is felt for new inhibitors which are highly effective in the treatment/prevention of multidrug resistance, in particular to chemotherapeutic drugs; particularly desired are new compounds having a high specificity to MDR1 and a low or null activity to other membrane transporters, so as not to interfere with the physiological operation of the other cell pumps which are not involved in drug resistance.

### SUMMARY

The invention relates to a selected family of compounds which are used as inhibitors of the activity of the multidrug resistance receptor 1 (MDR1). In fact, the Applicant found that such compounds are highly active towards the transporter MDR1, showing a medium to high selectivity to such transporter. They are useful in the treatment or prevention of multidrug resistance. The present compounds are described by the structural formula (I): 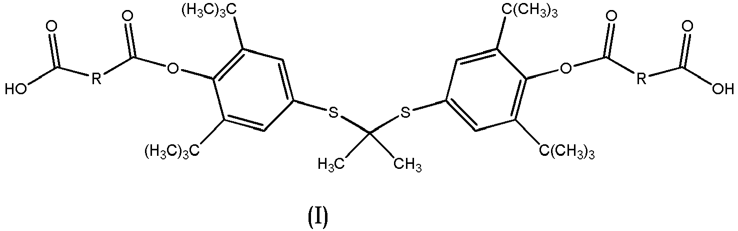 where R is a C₁-C₄ alkylene, optionally substituted with a C₁-C₃ alkyl.

Unlike other drugs used for treating the resistance to chemotherapeutic drugs, in particular drugs having a phenolic structure, the compounds of formula (I) have a low or null interference with the other membrane transporters of the *ATP binding cassette* (*ABC*) family, such as ABCA1 and ABCG1, which are physiologically involved in the metabolism of cholesterol; therefore, it does not give rise to phenomena of cell toxicity related to a non-specific, generalized inhibition of the membrane pumps; this allows it to be safely used at high doses and/or for long treatment periods.

### DESCRIPTION OF THE FIGURES

Fig. 1. Chemical structure of the molecule of formula (II) (AIF1).
Fig. 2. Quantifications of the levels of protein MDR1 on the plasma membrane in human carcinoma cell models (A) and assessment of MDR1 activity (B).
Fig. 3. Effect of AIF1 on the MDR1-mediated accumulation of fluorescent calcein in lung adenocarcinoma cells (A549) (A) and lung squamous-cell carcinoma (SKMES) (B).
Fig. 4. Effect of AIF1 on the ABCG2-mediated outflow of fluorescent Hoechst in human lung adenocarcinoma cells (H460).
Fig. 5. Effect of the compound AIF1 on the cell outflow process of cholesterol mediated by the membrane transporters ABCG1 (A) and ABCA1 (B).
Fig. 6. Effect of the compound AIF1 on the sensitivity of cancer cells of lung squamous-cell carcinoma (SKMES) to the chemotherapeutic drug doxorubicin.
Fig. 7. Quantification by confocal microscopy of intracellular doxurubicin in different times (A, B, C) in lung squamous-cell carcinoma (SKMES) cells in the presence and absence of AIF1.
Fig. 8. Measurement of the concentration of doxorubicin in the cancer cell after AIF1 treatment by liquid chromatography-tandem mass spectrometry (LCMS/ MS).
Fig. 9. Effect of AIF1 (II) and structural derivatives thereof IV (A), III, V (B), and VI, VII, VIII, IX (C), on the MDR1-mediated accumulation of fluorescent calcein in lung adenocarcinoma cells (A549).
Fig. 10: structure of the reference compounds of formula (IV)-(VI).
Fig. 11: structure of the reference compounds of formula (VII)-(IX).

### DETAILED DESCRIPTION

In the present formula (I) the group R is preferably unsubstituted; furthermore, R is preferably a C2-C3 alkylene, more preferably C3 alkylene.

In the compounds of formula (I), it is meant that the selected group R is the same in both positions: therefore, the molecule has a symmetric structure. Preferred compounds according to the invention have the following structures (II) and (III):

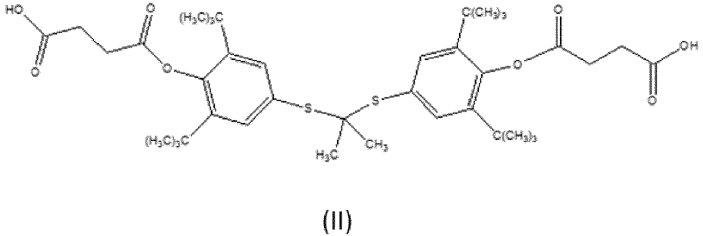

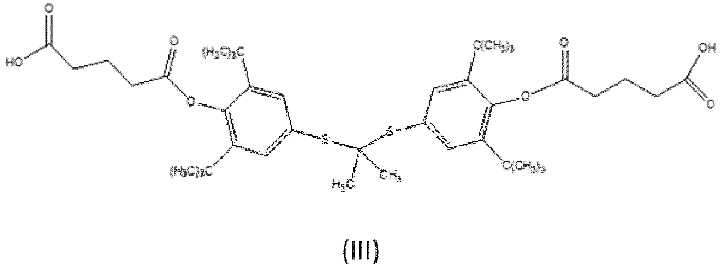

The compound of formula (II) is herein referred to also as AIF-1; the compound of formula (III) is herein referred to also as AIF-4.

The compounds of formula (I) can be synthetized according to known methods and by using currently available reagents. A preferred mode for the synthesis thereof is treating probucol with the anhydride of a dicarboxylic acid of formula HOOC-R-COOH, where R is as defined above, in the presence of sodium hydride (NaH) in tetrahydrofuran.

The molecule of formula (II) was described in the publication Bioorganic & Medicinal Chemistry Letters 12 (2002) 2545-48 as a biologically inactive synthesis intermediate; in said publication, it is synthetized starting from probucol, by a treatment with succinic anhydride in the presence of sodium hydride (NaH) in tetrahydrofuran.

Through original researches on cell models of human carcinoma, the Applicant has now found that the compounds of formula (I) selectively and effectively inhibit the activity of the MDR1 transporter. In particular, as experimentally shown for the compound AIF1, they significantly inhibit the outflow of the anti-cancer drug in various tumoral cell lines expressing high levels MDR1; the inhibition level is high, in the order of that reference standard PSC833. They affect the sensitivity of tumor cells to chemotherapeutic drugs which are substrates of the MDR1 pump: for example, the AIF1 treatment drastically reduced (by about 8 folds) the IC₅₀ value for doxorubicin, increasing the cell accumulation thereof: therefore, in the presence of the compounds of formula (I), tumor cells are made significantly less resistant to the drug transported by MDR1.

Unlike other phenolic compounds such as probucol, the present compounds have a low interference with the other *ATP binding cassette* transporters, ABCA1 and ABCG1.

The term "low interference" means that the compound of formula (I) has a low affinity to both the ABCA1 and ABCG1 transporters, or to only one of them, anyhow obtaining a low overall affinity towards the pair ABCA1 +ABCG1.

Consequently, the compounds of formula (I) have a low or null effect on the physiological outflow of cholesterol mediated by these transporters, representing an advantageous therapeutic option showing low toxicity in the treatment of multi-resistance.

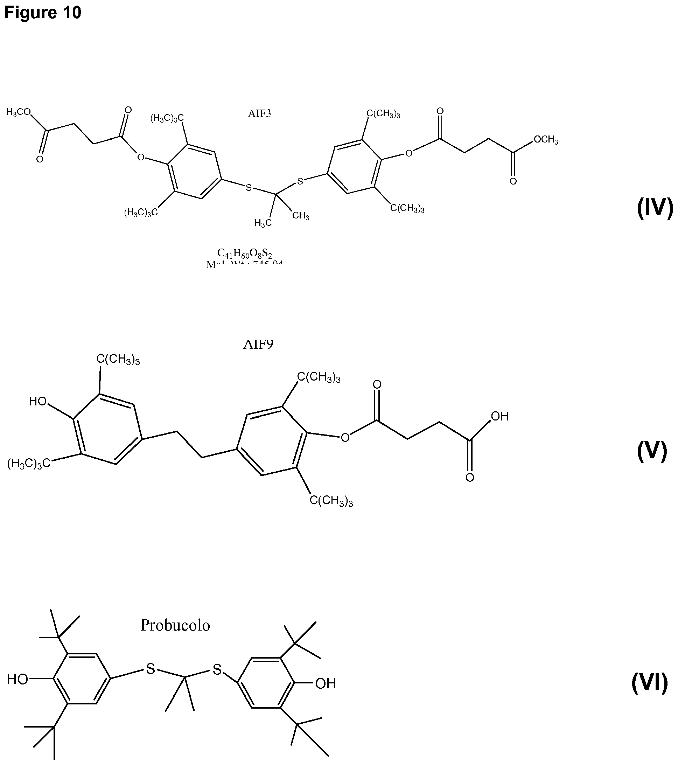

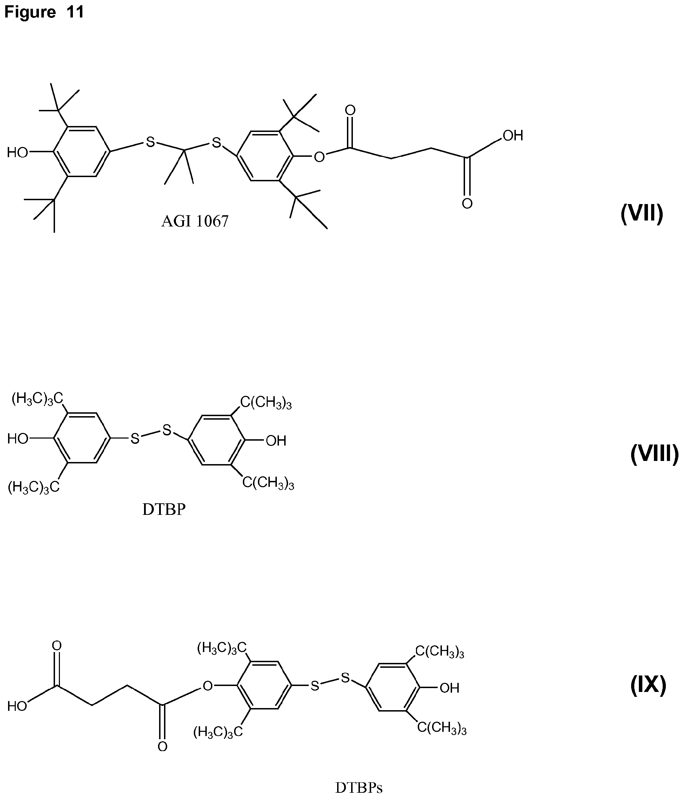

Finally, the Applicant assessed the effect of some structural modifications of the compounds of the invention (Figs. 10 and 11): all the thus modified reference derivatives gave rise to compounds which are inactive to MDR1: this highlights the importance of the combination of the structural elements constituting the compounds of formula (I).

Therefore, the compounds of formula (I) inhibit MDR1 in an effective and selective manner, thus being an excellent treatment of drug resistance, in particular restoring the sensitivity of cancer cells to the involved drugs, e.g., chemotherapeutic drugs, and concentrating them into the cancer cell.

In view of the above-mentioned new teachings, the present application claims the compounds of formula (I) or pharmaceutically acceptable salts thereof, for use in the treatment or prevention of drug resistance, where the drug giving rise to drug resistance is selected from chemotherapeutic drugs.

The invention includes pharmaceutical compositions wherein the compound of formula (I) or a salt thereof is formulated in the presence of suitable pharmaceutically acceptable excipients and with a drug giving rise to drug resistance, selected from chemotherapeutic drugs. The compositions may be in a solid, semisolid, or liquid form, according to the needs; the liquid forms may be, e.g., solutions or suspensions, emulsions, microemulsions; the solid forms may be, e.g., powders, granulates, tablets, capsules, microcapsules, suppositories; the semi-solid forms may be, e.g., creams, pastes, ointments, gels, or the like.

A further scope of the invention are the compounds of formula (I) (or the pharmaceutical compositions thereof) for use in a method of treating or preventing drug resistance to chemotherapeutic drugs. Among the resistances which can be advantageously treated according to the present invention, the resistances to anthracycline derivatives (e.g., doxorubicin, daunorubicin, epirubicin, idarubicin, etc.) can be mentioned. The compounds which are preferably used are those of formula (II) and (III), as described above.

The compounds of formula (I) are active on MDR1 in a dose-dependent manner; therefore, they can be used in a wide range of dosages as a function of the intensity of the desired effect; by way of non-limiting example, the dosage ranges between 10 and 500 mg/Kg, preferably between 20 and 300 mg/Kg, more preferably between 20 and 150 mg/Kg. By virtue of the specificity and low toxicity showed by the compounds of formula (I), it is possible to safely use also the high range of dosages. The compounds of formula (I) can be administered by any known administration mode, e.g., intravenous, intramuscular, oral, buccal, topical, transdermal, inhalation administration, etc., according to the treatment needs; the administration mode is preferably systemic. They can be administered individually, or in combination with further therapeutically active agents; among them, mention can be made of those drugs which give rise to drug resistance, and in particular the chemotherapeutic agents, for example, those having an anthracycline structure. Said further therapeutically active agents may be co-formulated with the compound of formula (I), or they can be administered separately in an administration regime wherein the chemotherapeutic drug is combined with the compound of formula (I). Therefore, a further scope of the invention consists in a kit of parts comprising, one separately from the other, a compound of formula (I) and a drug which gives rise to drug resistance, both being suitably formulated for the administration to the patient.

### EXPERIMENTAL PART

### 1. Experimental design and obtained results

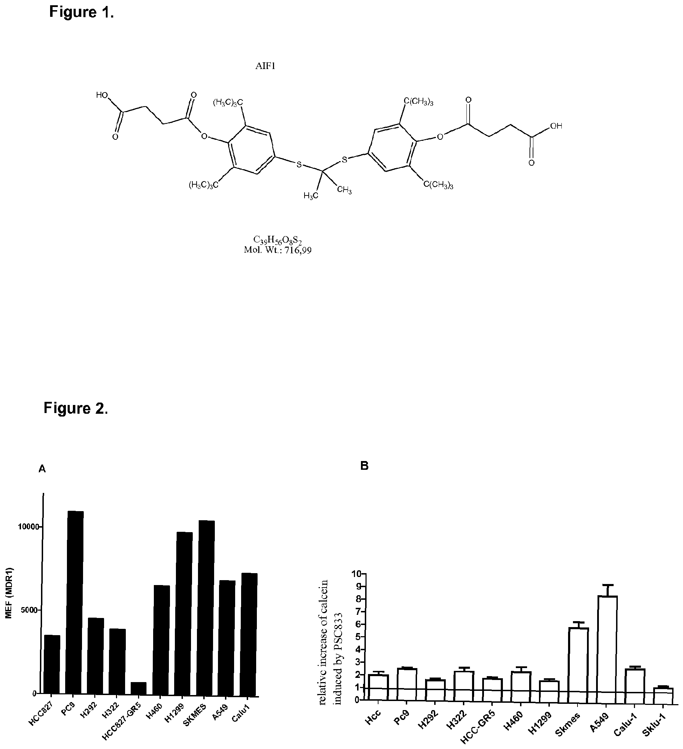

First, we quantified the content in protein MDR1 at the level of the plasma membrane of several cell lines of human lung carcinoma. As it can be noticed in Fig. 2, most of the tested cell lines have high levels of MDR1 on the plasma membrane. Next, we assessed the activity of the MDR1 pump in the same tumoral cell lines measuring the content in fluorescent calcein, MDR1 substrate, after treatment with PSC388, a known MDR1 inhibitor. The obtained results show that the cell models A549 and SKMES show a MDR1 activity which is significantly higher than the other tested models (Fig. 2A, 2B), suggesting that these represent the most suitable cell models for the assessment of the activity of the MDR1 pump.

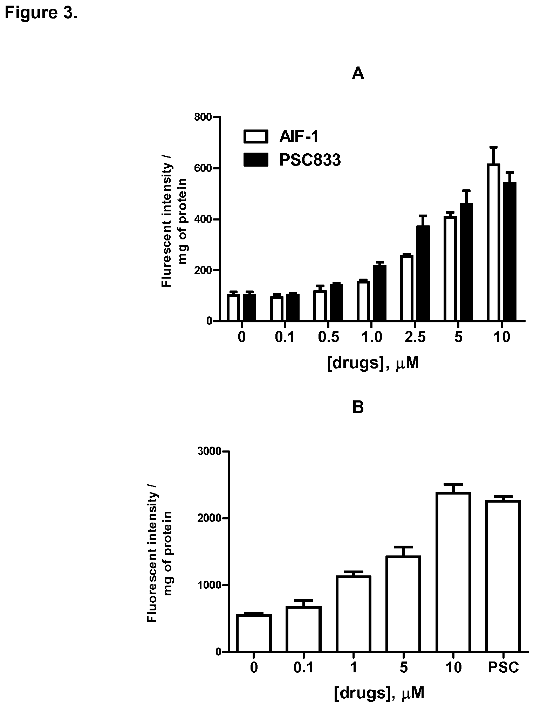

Then by using a cell model derived from human lung adenocarcinoma (A549), we assessed the ability of AIF1 of modulating the MDR1-mediated cell outflow of fluorescent calcein. As it can be noticed in Fig. 3A, the AIF1 treatment significantly and dose-dependent increases the intracellular concentration of calcein, suggesting an inhibitory effect of the compound on the MDR1 activity. Such a result was also confirmed when using a cell model of lung squamous-cell carcinoma (SKMES) expressing high levels of MDR1 on the plasma membrane (Fig. 3B). In both cases, AIF1 showed to be highly effective, similarly to the reference compound PSC833.

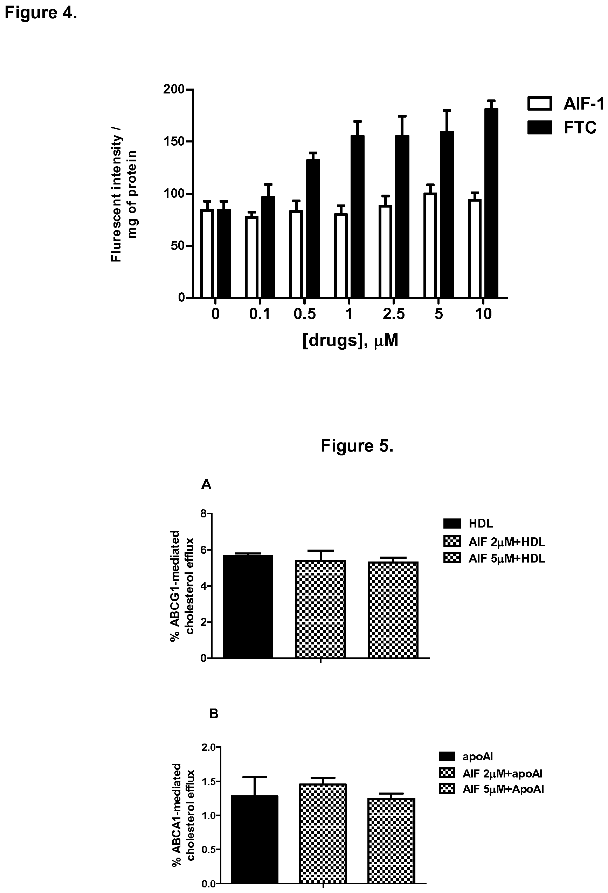

Then, we wanted to assess whether AIF1 was also active on another transporter involved in the resistance to chemotherapeutic drugs, the ATP-binding cassette transporter G2 (ABCG2), and with this aim we used a model of human lung adenocarcinoma (H460) over-expressing such receptor. The compound AIF1, unlike the known inhibitor of ABCG2 fumitrimorgin C, did not show any effect on the accumulation/extrusion of fluorescent Hoechst, a known substrate for ABCG2 (Fig. 4).

Furthermore, AIF1 was found inactive on other membrane receptors belonging to the family of the ABC transporters, which are physiologically involved in the cholesterol metabolism, and in particular the A1 (ABCA1) and G1 (ABCG1) ATP-binding cassette transporters. These transporters are involved in the first step of the reverse cholesterol transport (RCT) process, a physiological protective process whereby the excess cholesterol, which is present at the level of the peripheral tissues, is removed and transported to the liver for the clearance thereof from the body. The first step of this process is the cell outflow of the cholesterol from the macrophage of the artery wall to the lipoproteins circulating in the serum, such as apolipoprotein A-I (apoA-I) and the high-density lipoproteins HDLs, and it is mediated by the membrane transporters ABCA1 and ABCG1. As shown in Figs. 5A and 5B, AIF1 does not show any effect on the outflow of cholesterol mediated by the two transporters.

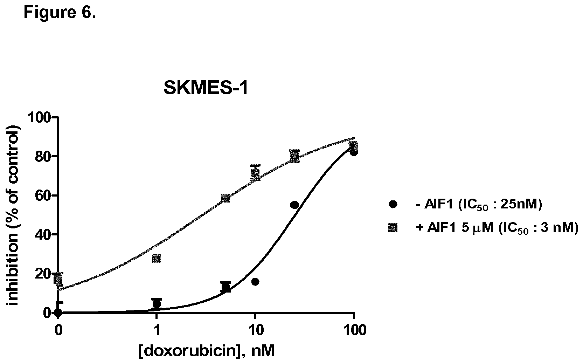

Subsequently, we assessed whether the new compound was able to affect the sensitivity of tumoral cells to doxorubicin, selected as the standard substrate of the MDR1 pump. The AIF1 treatment decreased by about 8-folds the IC₅₀ value for doxorubicin, indicating that, in the presence of AIF1, the tumoral cells are significantly less resistant to the drug (Fig. 6).

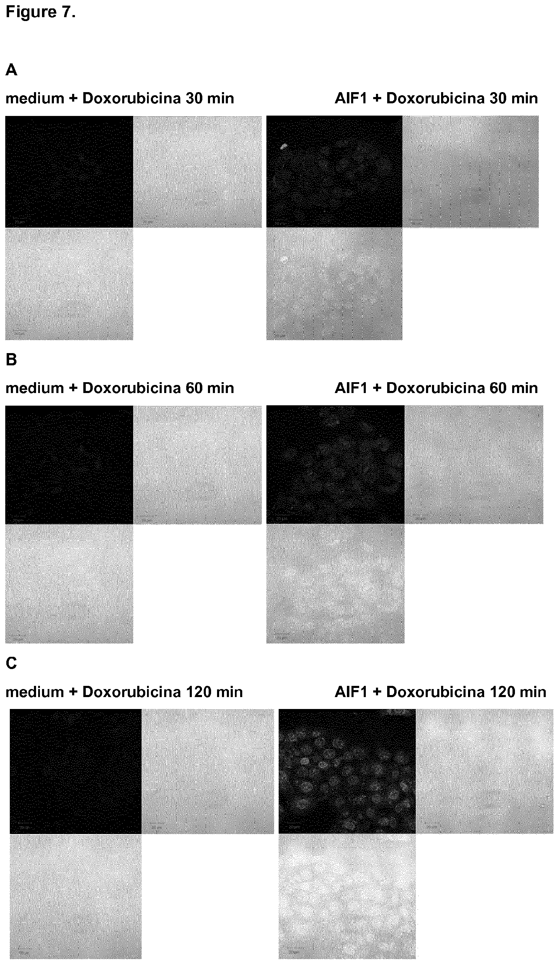

Then, we assessed the effect of the compound AIF1 on the intracellular content in doxorubicin by confocal microscope, exploiting the fluorescent signal emitted by the same doxorubicin. As it can be noticed from the images set forth in Fig. 7 (A, B, C), in the tumoral cells treated with doxorubicin in the presence of AIF1, already after 30 minutes the intracellular fluorescent signal was considerably increased with respect to the cells which had not been treated with the compound (Fig. 7A), indicating that AIF1 promotes the accumulation of doxorubicin and the obtainment of a higher intracellular concentration. The signal remains almost unaltered after 60 and 90 minutes of treatment with doxorubicin in the presence of AIF1 (Fig. 7B and 7C, respectively).

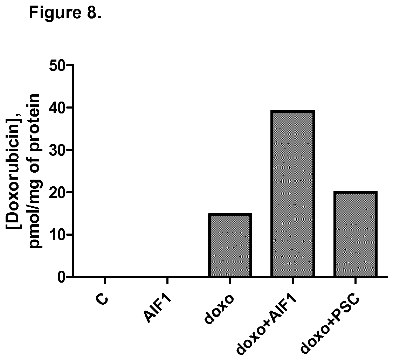

Then, we carried out an experiment of liquid chromatography-mass spectrometry (LC-MS/MS) in order to quantify the concentration of doxorubicin in tumoral cell after treatment with the compound AIF1. As it can be noticed in Fig. 8, the concentration of intracellular doxorubicin, expressed as pmol per mg protein, is significantly higher after AIF1 treatment compared to the untreated cells.

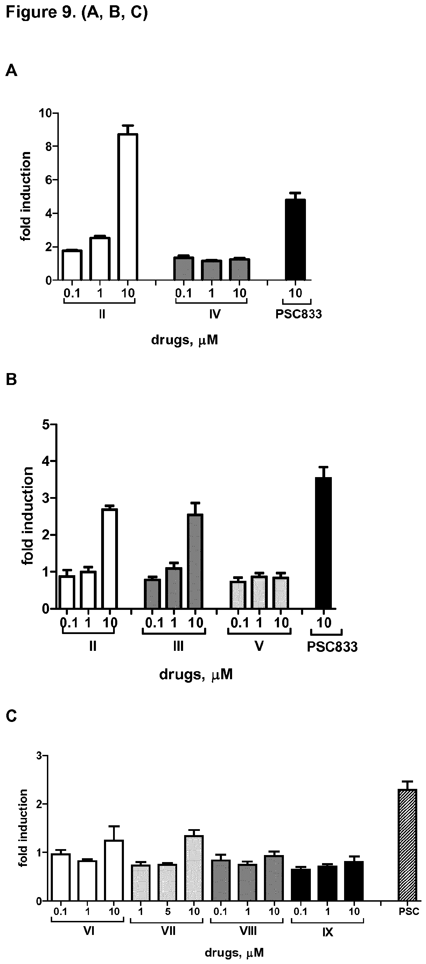

Furthermore, some reference derivatives of formula (IV)-(IX) (Figure 10 and 11) were synthetized, and the activity thereof towards the MDR1 pump were assessed in the same above-mentioned tumoral cell lines, measuring the content in fluorescent calcein; the obtained data, illustrated in Fig. 9 (A, B, C), gave a negative outcome, i.e., the tested compounds, although some of them are structurally related to the compounds of formula (I), did not show to be active towards the MDR1 pump.

### 2. Experimental methods

### 2.1 Methods of cell culture

The lung carcinoma cell lines A549 and SKMES are grown in RPMI, supplemented with 10% fetal calf serum (FCS), 2 mM glutamine (Gln), and antibiotics (Penicillin 100 UI/ml and Streptomycin 100 µg/ml). Cells are kept in a thermostat at a temperature of 37°C, in air saturated with water vapor, enriched with 5% CO₂.

### 2.2. Trypsinization and cell count

Once the growth medium has been removed, the cell monolayer is washed with PBS (phosphate buffer brine containing NaCl 8 g/l, KCl 0.2 g/l, Na₂HPO₄ •12 H₂OR 2.9 g/l, KH₂PO₄ 0.2 g/l) and then treated with of Trypsin 0.05% -EDTA 0.02% solution in PBS at 37° C. The suspension is then centrifuged for 4 minutes at 1800xg, and cells are counted by Bürker hemocytometer under light microscope.

### 2.3 Assessment of cell proliferation

Cell proliferation was assessed by a count of the cells in a Bürker chamber, after suitable dilutions. Those cells which were permeable to 0.1% of blue trypan, a dye which diffuses in the cells only when the membrane is damaged, were assessed as died.

### 2.4 Assessment of MDR1 and ABCG2 activity by loading with Calcein AM and Hoechst

Once the growth medium has been removed from the cells, a PBS washing at 37° C is carried out, and RPMI without phenol red containing antibiotics is added, 5% FCS, 10 mM HEPES and 2mM Glutamine in the presence/absence of Calcein AM or 1 µM Hoechst and possible inhibitors are added. At the end of the loading, two washings with cold PBS are carried out, RPMI without Calcein AM or Hoechst is added, and samples are read by using the fluorescence plate reader (Enspire, Perkin Elmer; λ of excitation: 360 nm; λ of emission: 465 nm). The value provided by the instrument is proportional to the intracellular concentration of Calcein AM and Hoechst. Finally, the cells are treated with 5% TCA, followed by 0.5 N NaOH to solubilize the cells. The protein content is determined by the Bradford method, and it is used for normalizing the uptake values of Calcein AM and Hoechst. The accumulation of Calcein or Hoechst is therefore expressed as the fluorescence intensity/mg proteins.

### 2.5 Protein dosage

Protein concentration is determined by the Coomassie Blue R250 staining assay (Bio-Rad). By using bovine serum albumin as the standard, protein concentration is determined by a spectrophotometer at a 595 nm wavelength.

### 2.6 Cytofluorimetric analysis of the ABCG2 and MDR1 levels expressed on the plasma membrane

5x105 cells were incubated for 1 hour at room temperature with anti-MDR1-PE or anti-ABG2-PE antibodies (BioLegend, London, UK), respectively. As an isotype control, for each studied line, 5x105 cells are incubated in parallel for 1 hour at room temperature with anti-IgG2-PE (BioLegend, London, UK). At the end of the incubation period, the cells were analyzed with a EPICS-XL cytofluorimeter (Beckman Coulter, Inc.)

The average fluorescence intensities (MFI) taken with the cytofluorimeter were then converted into equivalent units of fluorochrome (MEF) by using the FluoroSpheres 6-peak kit (Dako, CA, USA).

### 2.7 Measurement of the intracellular concentration of doxorubicin and AIF1

The intracellular content of Doxorubicin and AIF-1 may be determined by liquid chromatography-tandem mass spectrometry (LC-MS/MS) of samples extracted with absolute ethanol. In particular, after the treatment with the drugs, the culture medium is removed, and 3 washings of the cell monolayer with PBS are carried out. Then, the cells are incubated for ten minutes at room temperature with absolute ethanol, which is then analyzed by a LC-MS/MS technique. Values were normalized for the protein content assessed after solubilization of the cell monolayer with 0.5N NaOH and are expressed as pmoles/mg proteins.

### 2.8 LC-MS/MS

Doxorubicin and AIF-1 concentrations in the samples of cell extracts were determined by liquid chromatography-tandem mass spectrometry (LCMS/ MS) by using a mass spectrometer.

The samples extracted in ethanol were filtered on a 0.2 µm cellulose filter and analyzed by liquid chromatography-tandem mass spectrometry (LC-MS/MS).

Analyses were carried out by using a HP 1200 liquid chromatograph (Agilent Technologies, Palo Alto, CA, USA) provided with autosampler and coupled by electrospray interface (ESI) to a QTRAP 4000 triple quadrupole mass spectrometer (ABSCIEX, CA, USA). LC separation was obtained by using a Sunshell C18, 75 x 2.1 mm column, 2.6 □m particles (ChromaNik Technologies, Osaka, Japan), thermostated at a temperature of 25° C. The mobile phase is composed of a (A) water and (B) acetonitrile mixture with gradient elution: 0-2 min 5% B, 2-5 min 50% (B), 5-6 80% (B), 6-10 80% (B). Flow of the mobile phase 0.2ml/min, 20 µl injection volume. The system is controlled by the Analyst v1.6 software (ABSCIEX).

Source parameters: voltage of the capillary 4.5kV, source temperature 350°C, declustering potential 70V, entrance potential 10V.

The full scan mass spectra were recorded in the 250-900 m/z range, using a 0.1 stepsize and 1 s scan time in the positive ion mode for doxorubicin and atenolol (ATN, used as the internal standard) and in the negative ion mode for AIF1. The fragmentation spectrum for doxorubicin and AIF1 was taken in the production scan mode, selecting the protonated and deprotonated, respectively, molecular ion as the precursor ion, and recording the signal in the m/z 50-800 range, with a collisional energy (CE) ranging between 5 and 100 eV. The validation method and the quantitative analysis were carried out in the selected reaction monitoring (SRM) mode: doxorubicin m/z 544.5/397.3 (CE 18eV), 544.5/379 (CE 30 eV); AIF1 m/z715.7/515.5 (CE -25 eV); ATN (IS) m/z 267.3/145.3 (CE 33 eV). The calibration straight line was drawn in the range of 2.5-100ng/ml (doxorubicin) and 10-1000 ng/ml (AIF1).

### 2.9 Determination of the intracellular distribution of doxorubicin by confocal microscope

SKMES cells were seeded on cover glasses and incubated with culture medium in a thermostat for 24h. The day after cells were incubated with or without AIF-1 or PSC833 10 µM for 30 minutes before the treatment with 5 µM doxorubicin for 30 min, 60 minutes and 120 minutes, and at the end they were analyzed by confocal analysis.

Images were taken by using a confocal microscope (LSM 510 Meta scan integrated with a reversed microscope Carl Zeiss, Jena Germany), with a 43x/1.30 oil immersion lens.

Fluorescence of doxorubicin was excited with a 488nm argon laser and its emission was collected by a 530nm filter. During the analysis of the various samples, the confocal settings (excitation, laser power, detection range, and pinhole size) were kept unaltered.

### 2.10 ABCA1-mediated cholesterol outflow

Murine peritoneal macrophages were seeded in da 24-well plates, each containing 10⁶ cell/well in 10% RPMI of fetal bovine serum (FBS). After 24h, following a PBS washing, the cells were incubated with radiolabeled medium containing [3H]-cholesterol at a concentration of 2 µCi/ml in a medium supplemented with FBS 2% in the presence of the inhibitor of the esterifying enzyme ACAT (SAH58035) at a final concentration of 2 µg/ml in order to maintain all the intracellular cholesterol in the free form and the ABCA1 substrate in the outflow process. At the completion of the 24-hour staining, after the PBS washing, the cells were incubated with an equilibration medium composed of RPMI, 0.2% albumin, and the ACAT inhibitor. In this step, the expression of the ABCA1 protein on the plasma membrane was stimulated by adding 22-OH cholesterol and 9-cis-retinoic acid, which are agonists of the orphan nuclear receptors LXR/RXR (Liver X Receptor/Retinoic X Receptor) to the medium. At the end of the equilibration, cells were treated for two hours with medium at 0,2% BSA in the presence or absence of the compound AIF1. Cholesterol outflow was then promoted by apolipoprotein A-I at a concentration of 10µg/ml. After 4 hours, an aliquot of medium was collected from each well and transferred to a 0.45 µm filtering plate, so as to remove any cells suspended in the medium. The filtered medium was collected and transferred into a vial, together with the scintillation liquid for the subsequent reading at the β-counter for the measurement of the radioactivity present in the cultural medium, which is an index of the free cholesterol excreted by the cells. The outflow percentage is calculated on the total radioactivity which is present in the cell by the following formula: % outflow = (average cpm/total cpm of the monolayer) x 100. Each experimental condition was carried out in triplicates, and data was expressed as mean ± standard deviation.

### 2.11 ABCG1-mediated cholesterol outflow

In order to assess the ABCG1-mediated outflow, control hamster ovary cells CHO-K1 and CHO-L3 over-expressing the ABCG1 protein in Ham's medium F12 with 10% FBS were seeded in parallel in sterile 24-well plates. After 24h, both cell lines were labeled with 10% FBS medium containing 1µCi/ml [3H]-cholesterol. At the end of the labelling period (24h), both cell lines were washed with PBS and equilibrated during 90 minutes with medium supplemented with 0.2% albumin. At the end of the equilibration, the cells were treated in the absence or presence of our compound AIF1 during 2 hours, and incubated for the next 6 hours e with the cholesterol acceptors, HDLs, at a concentration of 12.5 µg/ml. The quantification of the tritiated cholesterol secreted by the cells in the medium was determined as described in the previous paragraph. In particular, the contribution of the ABCG1 transporter to the cholesterol outflow was calculated as the difference between the outflow percentages obtained from the cell line CHO-L3 over-expressing ABCG1 and those obtained from the control cell line CHO-K1. Each experimental condition was carried out in triplicates, and data was expressed as mean ± standard deviation.

## Claims

1. Pharmaceutical composition comprising a compound of formula (I) or a pharmaceutically acceptable salt thereof 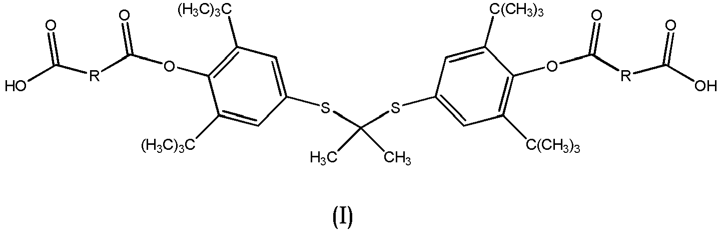 wherein R is a C₁-C₄ alkylene, optionally substituted with a C₁-C₃ alkyl,
in presence of one or more pharmaceutically acceptable excipients, the composition further comprising a drug which gives rise to drug resistance, said drug being selected from chemotherapeutic drugs.

2. Pharmaceutical composition according to claim 1, where R is a non-substituted C₂ or C₃ alkylene.

3. Pharmaceutical composition according to Claim 2, in a form selected from solution, suspension, emulsion, microemulsion, powder, granulate, tablet, capsule, microcapsule, suppository, cream, paste, ointment, gel.

4. Pharmaceutical composition according to Claims 1-3, where said chemotherapeutic drug has an anthracycline structure.

5. Kit of parts separately comprising a compound of formula (I) as described in claim 1-2, and a drug which gives rise to drug resistance, said drug being selected from chemotherapeutic drugs.

6. Compound of formula (I) according to Claims 1-2, for use in a method of treating or preventing resistance to chemotherapeutic drugs.

7. Compound for use according to Claim 6, where said chemotherapeutic drugs have anthracycline structure.

8. Compound for use according to Claims 6-7, in a form suitable for intravenous, intramuscular, oral, buccal, topical, transdermal or inhalation administration.

9. Compound for use according to Claims 6-8, administered at a dosage comprised between 10 and 500 mg/Kg,

10. Compound for use according to Claims 6-9, in combination with a drug which gives rise to drug resistance, said drug being selected from chemotherapeutic drugs, said combination being obtained via co-formulation or co-administration of said compound and said drug.

## Patentansprüche

1. Pharmazeutische Zusammensetzung, umfassend eine Verbindung der Formel (I) oder ein pharmazeutisches annehmbares Salz hiervon 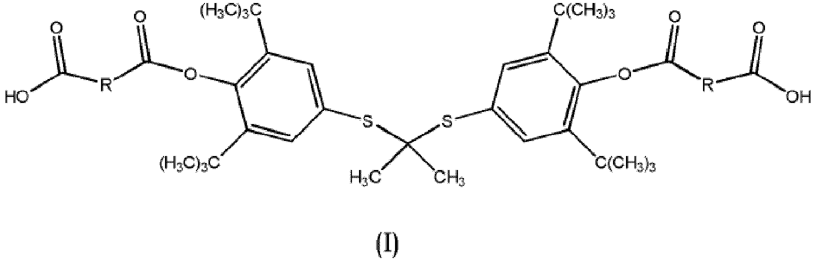 worin R ein wahlweise mit einem C₁-C₃-Alkyl substituiertes C₁-C₄-Alkylen ist,
in Gegenwart eines oder mehrerer pharmazeutisch annehmbarer Hilfsstoffe, wobei die Zusammensetzung weiterhin ein Arzneimittel umfasst, das zu Arzneimittelresistenz führt, wobei das Arzneimittel aus chemotherapeutischen Arzneimitteln gewählt ist.

2. Pharmazeutische Zusammensetzung nach Anspruch 1, wobei R ein nichtsubstituiertes C₂- oder C₃-Alkylen ist.

3. Pharmazeutische Zusammensetzung nach Anspruch 2 in einer Form, gewählt aus Lösung, Suspension, Emulsion, Mikroemulsion, Pulver, Granulat, Tablette, Kapsel, Mikrokapsel, Suppositorium, Creme, Paste, Salbe, Gel.

4. Pharmazeutische Zusammensetzung nach den Ansprüchen 1 - 3, wobei das chemotherapeutische Arzneimittel eine Anthracyclinstruktur besitzt.

5. Kit aus Teilen, separat umfassend eine Verbindung der Formel (I) wie in Ansprüchen 1 - 2 beschrieben, und ein Arzneimittel, das zu Arzneimittelresistenz führt, wobei das Arzneimittel aus chemotherapeutischen Arzneimitteln gewählt ist.

6. Verbindung der Formel (I) nach den Ansprüchen 1 - 2 zur Verwendung in einem Verfahren zur Behandlung oder Vermeidung von Resistenz gegenüber chemotherapeutischen Arzneimitteln.

7. Verbindung zur Verwendung nach Anspruch 6, wobei die chemotherapeutischen Arzneimittel Anthracyclinstruktur besitzen.

8. Verbindung zur Verwendung nach den Ansprüchen 6 - 7 in einer Form, geeignet für intravenöse, intramuskuläre, orale, bukkale, topische, transdermale oder Inhalationsverabreichung.

9. Verbindung zur Verwendung nach den Ansprüchen 6 - 8, verabreicht mit einer Dosis, die zwischen 10 und 500 mg/kg umfasst.

10. Verbindung zur Verwendung nach den Ansprüchen 6 - 9, in Kombination mit einem Arzneimittel, das zu Arzneimittelresistenz führt, wobei das Arzneimittel aus chemotherapeutischen Arzneimitteln gewählt ist, wobei die Kombination durch Co-Formulierung oder CoVerabreichung der Verbindung und des Arzneimittels erhalten wird.

## Revendications

1. Composition pharmaceutique comprenant un composé de formule (I) ou sel pharmaceutiquement acceptable de celui-ci 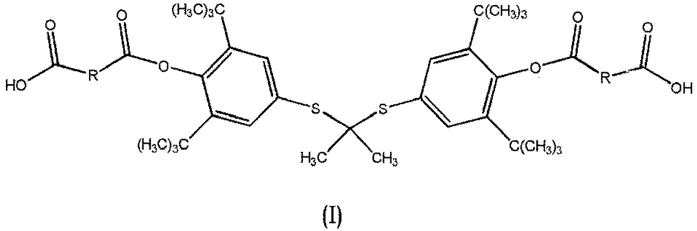 dans lequel R est un alkylène en C₁-C₄, optionnellement substitué par un alkyle en C₁-C₃,
en présence d'un ou plusieurs excipients pharmaceutiquement acceptables, la composition comprenant en outre un médicament qui provoque une résistance aux médicaments, ledit médicament étant choisi parmi les médicaments chimiothérapeutiques.

2. Composition pharmaceutique selon la Revendication 1, dans laquelle R est un alkylène en C₂ ou C₃ non substitué.

3. Composition pharmaceutique selon la Revendication 2, sous une forme choisie parmi une solution, une suspension, une émulsion, une microémulsion, une poudre, un granulé, un comprimé, une capsule, une microcapsule, un suppositoire, une crème, une pâte, une pommade, un gel.

4. Composition pharmaceutique selon les Revendications 1-3, où ledit médicament chimiothérapeutique a une structure anthracycline.

5. Kit comprenant séparément un composé de formule (I) tel que décrit dans les Revendications 1-2, et un médicament qui provoque une résistance aux médicaments, ledit médicament étant choisi parmi les médicaments chimiothérapeutiques.

6. Composé de formule (I) selon les Revendications 1-2, pour son utilisation dans une méthode de traitement ou de prévention de la résistance aux médicaments chimiothérapeutiques.

7. Composé pour son utilisation selon la Revendication 6, où lesdits médicaments chimiothérapeutiques ont une structure anthracycline.

8. Composé pour son utilisation selon les Revendications 6-7, sous une forme appropriée pour une administration intraveineuse, intramusculaire, orale, buccale, topique, transdermique ou par inhalation.

9. Composé pour son utilisation selon les Revendications 6-8, administré à une dose comprise entre 10 et 500 mg/kg.

10. Composé pour son utilisation selon les Revendications 6-9, en combinaison avec un médicament qui provoque une résistance aux médicaments, ledit médicament étant choisi parmi les médicaments chimiothérapeutiques, ladite combinaison étant obtenue par co-formulation ou co-administration dudit composé et dudit médicament.
